# EUROPEAN PATENT APPLICATION

(11) **EP 1 321 141 A1**
(43) Date of publication of application: **25.06.2003**
(21) Application number: 01963393.2
(22) Date of filing: 29.08.2001
(51) Int. Cl.: A61K 31/365, A61K 31/593, A61P 5/18, C07D 307/58, C07C 401/00

(54) **PARATHYROID HORMONE PRODUCTION INHIBITORS CONTAINING VITAMIN D3 DERIVATIVES**

(30) Priority: 30.08.2000 JP 2000260582
(71) Applicant: TEIJIN LIMITED, Osaka-shi Osaka 541-0054 (JP)
(72) Inventor: ISHIZUKA, Seiichi, Hino-shi, Tokyo 191-0065 (JP); MIURA, Daishiro, Hino-shi, Tokyo 191-0065 (JP); MANABE, Kenji, Hino-shi, Tokyo 191-0065 (JP); GAO, Qingzhi, Hino-shi, Tokyo 191-0065 (JP); SOGAWA, Ryo, Hino-shi, Tokyo 191-0065 (JP); TAKENOUCHI, Kazuya, Hino-shi, Tokyo 191-0065 (JP)
(74) Representative: Hallybone, Huw George
(86) International application number: JP0107431
(87) International publication number: WO02017911

(57) **Abstract**

An agent for suppressing the production of parathyroid hormone and a remedy for hyperparathyroidism containing a vitamin D₃ derivative described by the following general formula (1) 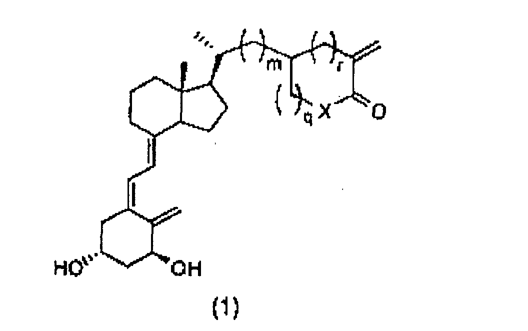 [in the formula, m is an integer of from 1 to 3, q is an integer of from 0 to 3, r is an integer of from 0 to 3 and X is carbon atom or oxygen atom, provided that 1≦q+r≦3] as an active ingredient.

## Description

### Technical Field

This invention relates to parathyroid hormone (hereafter referred to as "PTH") production inhibitors containing vitamin D₃ derivatives as the active ingredient. The invention further relates to remedies for hyperparathyroidism containing vitamin D₃ derivatives as the active ingredient.

### Background Arts

PTH is a polypeptide consisting of 84 amino acids and its main target organs are bone, cartilage and kidney. It is known that after bonding to the receptor of a target cell, PTH starts various intra- and inter-cellular cascades such as the promotion of the production of intracellular cyclic adenosine monophosphate (cAMP), the phosphorylation of intracellular proteins, the flow of calcium into a cell, the stimulation of the metabolic path of membrane phospholipids, the activation of intracellular enzyme and the secretion of lysosome enzyme. It is also known that the expression of PTH gene is subjected to suppressive control mainly with activated vitamin D₃ (Proc. Natl. Acad. Sci. U.S.A.), vol.89, pp.8097-8101, 1992). It is reported that the abnormality in the production amount of PTH in vivo causes various diseases. Examples of the diseases are primary hyperparathyroidism and secondary hyperparathyroidism accompanying to abnormal increase of PTH production.

The primary hyperparathyroidism is a systemic disease caused by the excessive PTH secretion from one or more parathyroid glands and about 90% of the patients are affected by parathyroid tumor. The secondary hyperparathyroidism is a disease developed by the excessive secretion of PTH caused by the metabolic disturbance of activated vitamin D, calcium and phosphorus of a patient of chronic renal failure resulting in the growth of parathyroid gland to exhibit resistance to 1α,25-dihydroxyvitamin D₃ of physiological concentration and further progress hyperplacia. There are many cases accompanying ostealgia and arthralgia owing to the increase of bone resorption by excessive PTH. Further, the disease sometimes develops symptoms other than bone part such as ectopic calcification of soft tissue and arterial wall caused by hypercalcemia and hyperphosphatemia.

The production mechanism of secondary hyperparathyroidism and the pathologic physiology of the parathyroid gland are being gradually cleared recently and the findings are influencing the therapeutic method. The possible production mechanisms of secondary hyperparathyroidism are the trade-off theory, the activation disturbance of vitamin D in the kidney, the resistance to 1α,25-dihydroxyvitamin D₃, the abnormality of sensitivity to calcium, the direct action of phosphorus, etc.

The most important point among these factors is the accumulation of phosphorus by the lowering of the renal function to increase the load of phosphorus on the uriniferous tubule and inhibit the activity of 1α-hydroxylase. The concentration of 1α,25-dihydroxyvitamin D₃ in serum is lowered by the continuation of the lowered state of renal function to cause hypocalcemic state by the calcium absorption disorder in small intestines, etc., and the hyperphosphatemia according to the lowering of phosphorus secretion from the kidney. Continuing hypocalcemia is supposed to promote the secretion of PTH and develop the secondary hyperparathyroidism.

Accordingly, the preferable treatment for the secondary hyperparathyroidism is the administration of a compound effective for suppressing the secretion of PTH from parathyroid gland or a compound having parathyroid cell proliferation suppressing action or the administration of remedies effective for increasing a 1α,25-dihydroxyvitamin D₃ receptor or calcium sensing receptor which have been confirmed to be lowered in the parathyroid cell. Known compounds having the above characteristics are 1α-hydroxyvitamin D₃ and 1α,25-dihydroxyvitamin D₃ and these compounds are administered as remedies for the secondary hyperparathyroidism to achieve extremely high effectiveness.

However, the number of 1α,25-dihydroxyvitamin D₃ receptor in the parathyroid gland is decreased in the patients with chronic renal insufficiency administered with such activated vitamin D₃ preparation for a long period and it has been difficult to suppress the proliferation of parathyroid cell and the hypersecretion of PTH by the administration of the ordinary amount of activated vitamin D₃ preparation.

Slatopolsky, et al. have succeeded in the suppression of PTH secretion from parathyroid gland by the intermittent intravenous injection of a large amounts of 1α,25-dihydroxyvitamin D₃ (J. Clin. Invest., vol.74, pp.2136-2143, 1984). This is known as activated vitamin D pulse therapy.

As the theoretical basis of the therapy, it has been suggested that activated vitamin D₃ increases 1α,25-dihydroxyvitamin D₃ receptor lowered in the parathyroid gland of a patients with chronic renal insufficiency (J. Clin. Invest. vol.86, pp.1968-1975, 1990) and increases calcium sensing receptor (Am. J. Physiol. vol.270, pp.F454-F460, 1996).

However, the activated vitamin D pulse therapy is liable to cause hypercalcemia by the administration of a large amounts of 1α,25-dihydroxyvitamin D₃ preparation. Accordingly, a 1α-hydroxyvitamin D₂ preparation (International Patent Application WO96/31215) a 19-nor-1α,25-dihydroxyvitamin D₂ preparation (International Patent Application WO97/02826) and a 22-oxa-1α,25-dihydroxyvitamin D₃ preparation (Japanese Laid-open Patent Application (hereinafter referred to as JP-A) 3-7231) having calcium metabolic activities weaker than that of the 1α,25-dihydroxyvitamin D₃ preparation are being used at present as remedies for vitamin D-resistant secondary hyperparathyroidism.

Although these vitamin D₃ derivatives have weak calcium metabolic activity compared with 1α,25-dihydroxyvitamin D₃, these derivatives still remain weak activity which causes insufficient separation of PTH production suppressing action and calcium metabolic activity to often cause hypercalcemia as a side action (Nephrol. Dial. Transport vol.11, pp.121-129, 1996). Therefore, it is hard to say from the viewpoint of side effects that the therapy using these preparations is sufficiently satisfactory. Accordingly, more effective therapeutic effect is expectable by a medicine which strongly suppresses PTH secretion without causing hypercalcemia.

The vitamin D₃ derivative to be used in the present invention can be synthesized by the methods described in the International Patent Application WO96/33716 and the International Patent Application WO00/24712. These compounds are known to have bone resorption inhibiting activity and osteogenesis promoting activity (International Patent Application WO95/33716) and neutrophil infiltration suppressing activity (International Patent Application WO00/24712). Furthermore, the specification of the JP-A 11-35470 discloses that the derivative has PTH production promoting activity.

### Disclosure of the Invention

The purpose of the present invention is to provide a PTH production suppressing agent free from actions to increase the calcium concentration in the serum. Another purpose of the present invention is to provide remedies for hyperparathyroidism free from actions to increase the calcium concentration in the serum.

The purposes of the present invention can be achieved by a PTH production suppressing agent containing a vitamin D₃ derivative expressed by the following general formula (1) 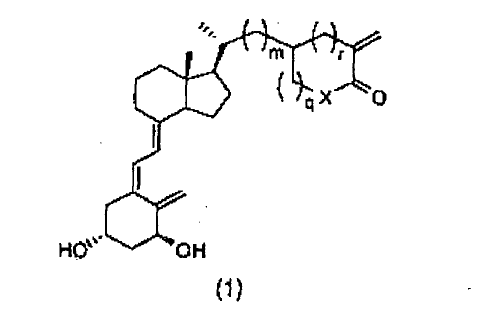 [in the formula, m is an integer of from 1 to 3, q is an integer of from 0 to 3, r is an integer of from 0 to 3 and X is carbon atom or oxygen atom, provided that 1≦q+r≦3] as an active ingredient. Further, the purpose is achieved by the use of the PTH production suppressing agent as a remedy for diseases caused by the promotion of PTH production, especially hyperparathyroidism.

The specification of the present invention describes that these compounds can strongly suppress the increased blood PTH level of vitamin D deficient animals without influencing the serum calcium level. The result seems to have contradiction with the PTH production promoting effect described in the abovementioned JP-A 11-35470, however, the contradiction can be explained as follows.

The vitamin D₃ derivative used in the present invention has both antagonistic action and agonistic action and, in the specification of JP-A 11-35470, the vitamin D₃ derivative used in the present invention developed the antagonistic action and promoted the production of PTH because the experiment in the specification was carried out by using normal mouse administered with 1α,25-dihydroxyvitamin D₃. Contrary, the experimental system of the present invention used a vitamin D deficient animal free from 1α,25-dihydroxyvitamin D₃ and, accordingly, the vitamin D₃ derivative of the present invention developed agonistic action to suppress the production of PTH.

Since the physiological state of a patient of hyperparathyroidism is similar to that of a vitamin D deficient animal, the vitamin D₃ derivative of the present invention is useful as a remedy for hyperparathyroidism. The antagonistic action of the vitamin D₃ derivative of the present invention is described also in the specifications of JP-A 11-5787 and International Patent Application WO00/24712.

### Best Mode for Carrying out the Invention

Among the vitamin D₃ derivatives expressed by the above formula (1) and used in the present invention, preferable derivatives are those of the formula wherein the term m is 1 or 2. The preferable combinations of the terms m, q, r and X are described in the Table 1 and the compounds of No. 11, 13, 16, 21, 23 and 26 are especially preferable. When the compound in the Table contains asymmetric carbon atom in the structure, the compound include both of (S) configuration and (R) configuration unless otherwise mentioned.

**Table 1**

| Compound No. | m | q | r | X |
|---|---|---|---|---|
| 11 | 1 | 0 | 1 | Oxygen atom |
| 12 | 1 | 1 | 1 | Oxygen atom |
| 13 | 1 | 0 | 1 | Carbon atom |
| 14 | 1 | 0 | 2 | Carbon atom |
| 15 | 1 | 0 | 3 | Carbon atom |
| 16 | 1 | 1 | 0 | Carbon atom |
| 17 | 1 | 2 | 0 | Carbon atom |
| 18 | 1 | 3 | 0 | Carbon atom |
| 21 | 2 | 0 | 1 | Oxygen atom |
| 22 | 2 | 1 | 1 | Oxygen atom |
| 23 | 2 | 0 | 1 | Carbon atom |
| 24 | 2 | 0 | 2 | Carbon atom |
| 25 | 2 | 0 | 3 | Carbon atom |
| 26 | 2 | 1 | 0 | Carbon atom |
| 27 | 2 | 2 | 0 | Carbon atom |
| 28 | 2 | 3 | 0 | Carbon atom |

The hyperparathyroidism is e.g. primary or secondary hyperparathyroidism caused by the promotion of PTH production.

A PTH production suppressing agent or a remedy for hyperparathyroidism containing the above compound as an active ingredient can be prepared in the form of pharmaceutical preparation by using the compound as an active ingredient and forming in the form of a peroral agent or an injection such as soft capsule, hard capsule, tablet and syrup by conventional method using a proper excipient. In the case of administering the compound once or plural times a day as an agent for suppressing the production of PTH or a remedy for hyperparathyroidism, it is preferable to use the compound as a peroral agent. An injection capable of achieving temporarily high blood concentration is preferable for a pulse therapy using the compound as a PTH production suppressing agent or a remedy for hyperparathyroidism.

The excipient to be used in the present invention for a liquid agent or a parenteral agent is, for example, vegetable oils, mineral oils, white petrolatum, branched-chain fats or oils and high-molecular weight alcohols. Preferable excipients among the above substances are, for example, vegetable oils such as cottonseed oil, corn oil, coconut oil and almond oil, especially preferably triglyceride of a medium-chain fatty acid.

Examples of preferable excipients for solid agent are cellulose derivatives such as crystalline cellulose, hydroxypropylcellulose, hydroxypropyl methylcellulose and methyl cellulose, polyvinyl pyrrolidone, dextrin, cyclodextrin, casein, lactose, mannitol and gelatin.

The amount of the active ingredient in the suppressing agent or the remedy of the present invention is determined according to the state of the disease and is generally from 0.00004 to 0.2% by weight, preferably from 0.0001 to 0.1% by weight.

The administration dose of the active ingredient is also dependent upon the state of the disease and it is generally from 0.1 to 1,000 µg/day/head, preferably from 1 to 100 µg/day/head or thereabout. The dosing frequency is usually once to thrice per day. The pharmaceutical preparation is preferably prepared in a manner to satisfy the above conditions.

The usefulness of the present invention has been shown, as described concretely in the Examples, by experiments using rats having promoted PTH secretion in a vitamin D deficient state. Namely, it has been found that the PTH concentration in serum rapidly decreases by the administration of the vitamin D₃ derivative shown by the above formula (1) (compound 11 (23S isomer)) to a vitamin D deficient rat.

The blood calcium level increasing activity of the vitamin D₃ derivative expressed by the above formula (1) (compound 11 (23S isomer)) is about 1/377 compared with 1α,25-dihydroxyvitamin D₃ revealing extremely weak activity of the derivative. Accordingly, the separation of the concentration to suppress the production of PTH from the concentration to develop the blood calcium level increasing action is realized in the vitamin D₃ derivative expressed by the above formula (1) and there is no development of hypercalcemia by the use of the derivative in contrast with conventional vitamin D preparation.

It is concluded from the above results that the vitamin D₃ derivatives expressed by the above formula (1) are useful as a PTH production suppressing agent and a remedy for hyperparathyroidism.

### Examples

### Example 1

### Action of (23S)-25-dehydro-1α,25-dihydroxyvitamin D₃-26,23-lactone (Compound No.11 (23S isomer) on the production of PTH in vitamin D deficient rat with time

(1) Male Wistar rats of 4 weeks old were purchased from Japan SLC (SLC, Shizuoka prefecture). The rats were put into wire cages three for each cage and bred under the condition of 23 ± 1°C and 55 ± 10% humidity by allowing the rats to free ingestion of a vitamin D deficient feed for animal breeding (Ca, 0.0036%; P, 0.3%; Harlan Teklad Research Diet, Madison, WI, U.S.A.) and drinking water (well water treated with 0.4% ± 0.2 ppm hypochlorite) for 7 weeks. The number of animals were five for one group.
(2) As shown in the Table 2, the negative control group (Group 1) was administered with a solvent (5% ethanol/0.1% Triton X-100/physiological saline solution) and the positive control group (Group 2) was administered with 0.5 µg/kg of 1α,25-dihydroxyvitamin D₃ by intravenous administration.
(3) As the group administered with the vitamin D₃ derivative, the group (Group 3) was administered with the compound 11 (23S isomer) at a dose of 50 µg/kg by intravenous administration. The volume of administered liquid was 2 mL/kg.
(4) The blood was collected from the descending abdominal aorta under anesthesia with ether 4, 8, 24, 48 and 72 hours after administration, serum was separated by the established method and the PTH concentration and the calcium concentrations in the serum were measured. The measurement of PTH concentration was carried out by using the radioimmunoassay kit for the measurement of rat PTH produced by Immutopics(San CA), and the calcium concentration was measured by the OCPC method (Am. J. Clin. Pathol), vol.45, pp.290-296, 1966) using the Type-7070 automatic analyzer manufactured by Hitachi, Ltd.
(5) The results are shown in the Table 2.

**Table 2**

| Change of PTH concentration and calcium concentration in serum with time after the administration of the Compound 11 (23S isomer) to vitamin D deficient rats | | | | | |
|---|---|---|---|---|---|
| Group | Compound | Administration Rate (mg/kg) | Time (hrs) | PTH cone. (pg/ml serum) | Calcium conc.(mg/100ml serum) |
| 1 | Solvent (negative control) | - | 0 | 512±46 | 4.93±0.12 |
| 2 | 1α,25-dihydroxyvitamin D₃ (positive control) | 0.5 | 4 | 458±38 | 5.33±0.09* |
| | | 0.5 | 8 | 399±2*** | 6.00±0.17*** |
| | | 0.5 | 24 | 325±31*** | 5.87±0.22*** |
| | | 0.5 | 48 | 488±43 | 5.48±0.15** |
| | | 0.5 | 72 | 555±67 | 5.10±0.18 |
| 3 | Compound 11 (23S isomer) | 50 | 4 | 354±65*** | 5.03±0.13 |
| | | 50 | 8 | 330±72*** | 5.53±0.12** |
| | | 50 | 24 | 319±1*** | 4.82±0.18 |
| | | 50 | 48 | 436±51** | 4.74±0.13 |
| | | 50 | 72 | 528±38 | 4.88±0.20 |
| | Normal rat of same week old | - | - | 56±17 | 10.21±0.06 |
| · The experimental results are shown by the mean ± standard error (the number of experiments n=5). | | | | | |
| · The statistical significance test of the experimental data was carried out by the Dunnet method and significant difference to the negative control group was obtained at a significance level of * : p<0.05, ** : p<0.01 and *** : p<0.001. | | | | | |

The intravenous administration of 1α,25-dihydroxyvitamin D₃ at a dose of 0.5 µg/kg showed the lowering of the PTH concentration in serum as early as 4 hours after the administration of 1α,25-dihydroxyvitamin D₃, and the concentration was gradually lowered after 8 hours and 24 hours and showed the lowest level after 24 hours. The level was gradually increased from the lowest level after 48 hours and 72 hours and returned to the concentration comparable to that of the negative control group (Group 1) after 72 hours.

The change of the calcium concentration in serum with time was completely adverse to that of the PHT concentration. The calcium concentration in serum began to increase as early as 4 hours after the administration of 1α,25-dihydroxyvitamin D₃, reached the maximum level after 8 hours, gradually lowered thereafter and returned to nearly the same level as the negative control group (Group 1) 72 hours after the administration.

On the other hand, the intravenous administration of the compound 11 (23S isomer) at a dose of 50 µg/kg significantly lowered the PTH concentration in serum as early as 4 hours after the administration and the concentration was lowered to the lowest level 24 hours after the administration, gradually increased and returned to the level of the negative control group after 72 hours. In this case, the calcium concentration in serum was increased temporarily 8 hours after the administration of the compound 11 (23S isomer) and was absolutely kept constant at the other time. The increase of the serum calcium concentration 8 hours after the administration is the result of the administration of the compound 11 (23S isomer) at a dose of 50 µg/kg, and there was absolutely no increase of the serum calcium concentration by the intravenous administration at a dose of 10 µg/kg and the PTH concentration was significantly lowered at the dose.

Accordingly, it has been cleared that, in contrast with 1α,25-dihydroxyvitamin D₃, the compound 11 (23S isomer) is effective for lowering the PTH concentration in serum without changing the calcium concentration in serum.

### Example 2

### Change of serum PTH concentration and serum calcium concentration of vitamin D deficient rat 8 hours after the administration of (23S)-25-dehydro-1α-dihydroxyvitamin D₃-26,23-lactone (Compound No.11 (23S isomer) at various concentrations

(1) The experimental animals, the breeding conditions, etc., of the experiment were similar to those of the Example 1.
(2) As shown in the Table 3, the negative control group (Group 1) was administered with a solvent (5% ethanol/0.1% Triton X-100/physiological saline solution) and the positive control group (Group 2) was administered with 0.25 µg/kg of 1α,25-dihydroxyvitamin D₃ by intravenous administration.
(3) As the group administered with the vitamin D₃ derivative, the group (Group 3) was administered with the compound 11 (23S isomer) at a dose of 2 µg/kg, 10 µg/kg and 50 µg/kg by intravenous administration. The volume of administered solution was 2 mL/kg.
(4) The blood was collected from the descending abdominal aorta under anesthesia with ether 8 hours after administration, serum was separated by the established method and the PTH concentration and calcium concentration in the serum were measured by the procedures similar to those of the Example 1.
(5) The results are shown in the Table 3.

**Table 3**

| Change of serum PTH concentration and serum calcium concentration of vitamin D deficient rats 8 hours after the administration of the Compound 11 (23S isomer) at various concentrations | | | | | |
|---|---|---|---|---|---|
| Group | Compound | Administration Rate (mg/kg) | Time (hrs) | PTH conc. (pg/ml serum) | Calcium conc. in serum (mg/100ml serum) |
| 1 | Solvent (negative control) | - | 8 | 546±35 | 4.67±0.23 |
| 2 | 1α,25-dihydroxyvitamin D₃ (positive control) | 0.25 | 8 | 459±14* | 5.50±0.17*** |
| 3 | Compound 11 (23S isomer) | 2 | 8 | 488±47* | 4.52±0.18 |
| | | 10 | 8 | 385±65*** | 4.45±0.05 |
| | | 50 | 8 | 319±56*** | 5.10±0.10* |
| | Normal rat of same week old | - | - | 56±17 | 10.21±0.06 |
| · The experimental results are shown by the mean ± standard error (the number of experiments n=5). | | | | | |
| · The statistical significance test of the experimental data was carried out by the Dunnet method and significant difference to the negative control group was obtained at a significance level of *: p<0.05, ** : p<0.01 and *** : p<0.001. | | | | | |

The serum PTH concentrations of the group (Group 3) administered with the compound 11 (23S isomer) were lowered depending upon the administration dose by the administration at the dose of 2 µg/kg, 10 µg/kg and 50 µg/kg compared with the negative control group (Group 1). It is known that 1α,25-dihydroxyvitamin D₃ also lowers dose-dependently the PTH concentration in serum and, in the present experiment, serum PTH concentration was significantly lowered by the administration of 1α,25-dihydroxyvitamin D₃ to the positive control group (Group 2). The serum PTH concentration lowering action of the compound 11 (23S isomer) was judged from the results to be about 1/12 of the action of 1α,25-dihydroxyvitamin D₃. The characteristic feature of the compound 11 (23S isomer) is the strong suppression of the PTH production at a dose not to cause the increase of serum calcium concentration in contrast with 1α,25-dihydroxyvitamin D₃ which increases the serum calcium concentration simultaneously with the lowering of the serum PTH concentration.

### Example 3

### Calcium metabolic activity of vitamin D deficient rats 8 hours after the administration of (23S)-25-dehydro-1α,25-dihydroxyvitamin D₃-26,23-lactone (compound No.11 (23S isomer)) at various concentrations

(1) The experimental animals, the breeding conditions, etc., of the experiment were similar to those of the Example 1.
(2) As shown in the Table 4, the negative control group (Group 1) was administered with a solvent (5% ethanol/0.1% Triton X-100/physiological saline solution) and the positive control group (Group 2) was administered with 1α,25-dihydroxyvitamin D₃ at a dose of 0.1 µg/kg, 0.5 µg/kg and 2.5 µg/kg by intravenous administration.
(3) As the group administered with the vitamin D₃ derivative, the group (Group 3) was administered with the compound 11 (23S isomer) (Group 3) at a rate of 10 µg/kg, 50 µg/kg and 250 µg/kg by intravenous administration. The volume of administered solution was 2 mL/kg.
(4) The blood was collected from the descending abdominal aorta under anesthesia with ether 8 hours after administration, serum was separated by the established method and the calcium concentration in the serum were measured by a procedure similar to the Example 1. The increase of serum calcium concentration observed by the experiment shows the bone resorption activity.
(5) After collecting the blood from the descending abdominal aorta under anesthesia with ether 8 hours after administration, the duodenum was extracted and the calcium absorption activity of the intestinal canal was determined by inverted gut sac method described in the Am. J. Physiol., vol.216, pp.1351-1359, 1969..
(6) The results are shown in the Table 4.

**Table 4**

| Calcium metabolic activity of vitamin D deficient rats 8 hours after the administration of the Compound 11 (23S isomer) at various concentrations | | | | | |
|---|---|---|---|---|---|
| Group | Compound | Administration Rate (mg/kg) | Time (hrs) | Intestinal calcium absorption (pg/ml serum) | Calcium conc. in serum (mg/100ml serum) |
| 1 | Solvent (negative control) | - | 8 | 1.86±0.08 | 4.73±0.18 |
| 2 | 1α,25-dihydroxyvitamin D₃ (positive control) | 0.1 | 8 | 2.66±0.09*** | 5.30±0.10** |
| | | | | | |
| | | 0.5 | 8 | 3.23±0.24*** | 5.90±0.40*** |
| | | | | | |
| | | 2.5 | 8 | 3.94±0.43*** | 6.17±0.07*** |
| 3 | Compound 11 (23S isomer) | 10 | 8 | 1.89±0.04 | 4.63±0.09 |
| | | 50 | 8 | 2.32±0.21* | 5.43±0.15** |
| | | 250 | 8 | 2.53±0.10*** | 5.63±0.07*** |
| · The experimental results are shown by the mean ± standard error (the number of experiments n=5). | | | | | |
| · The statistical significance test of the experimental data was carried out by the Dunnet method and significant difference to the negative control group was obtained at a significance level of *: p<0.05, ** : p<0.01 and ***: p<0.001. | | | | | |

The intestinal calcium absorption promoting activity and serum calcium concentration increasing activity, i.e. the bone resorption promoting activity of the negative control group (Group 1) were dose-dependently developed by the administration of 1α,25-dihydroxyvitamin D₃ at a dose of from 0.1 µg/kg to 2.5 µg/kg. In contrast with the above case, the activity was absolutely unobservable on the group (Group 3) administered with the compound 11 (23S isomer) at a dose of 10 µg/kg and weak intestinal calcium absorption promoting activity and serum calcium concentration increasing activity, i.e. the bone resorption promoting activity were observed at the administration dose of from 50 µg/kg to 250 µg/kg. However, the activities were about 1/1,400 and 1/377, respectively, compared with 1α,25-dihydroxyvitamin D₃. Furthermore, the activity caused by the compound 11 (23S isomer) was observable only at 8 hours after the administration and was absolutely unobservable at 4 hours and 24 hours after the administration. The result shows that the calcium metabolism activity caused by the compound 11 (23S isomer) is extremely weak compared with 1α,25-dihydroxyvitamin D₃.

It has been cleared from the results of the Examples 1, 2 and 3 that the vitamin D₃ derivative to be used in the present invention suppresses the PTH synthesis in parathyroid gland and quickly lowers the PTH concentration in serum without increasing the serum calcium concentration. This finding shows the usefulness of the vitamin D₃ derivative used in the present invention as a remedy for hyperparathyroidism caused by the promotion of PTH secretion.

### Field of Industrial Utilization

The remedy containing a vitamin D₃ derivative as an active ingredient and provided by the present invention can be used as an agent for suppressing the production of PTH or an agent for the treatment of hyperparathyroidism. These PTH production suppressing agent and hyperparathyroidism treating agent can be administered for example as an orally administrable agent or an injection for pulse therapy.

## Claims

1. An agent for suppressing the production of parathyroid hormone containing a vitamin D₃ derivative expressed by the following formula (1) 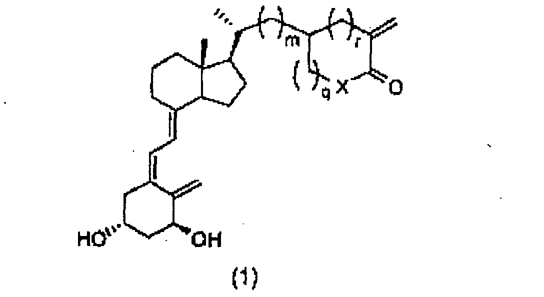 [in the formula, m is an integer of from 1 to 3, q is an integer of from 0 to 3, r is an integer of from 0 to 3, and X is carbon atom or oxygen atom, provided that 1≦q+r≦3] as an active ingredient.

2. A remedy for hyperparathyroidism containing the vitamin D₃ derivative expressed by the above formula (1) as an active ingredient.

3. A parathyroid hormone production suppressing agent described in the Claim 1 wherein m of the formula (1) is 1 or 2.

4. A remedy for hyperparathyroidism described in the Claim 2 wherein m of the formula (1) is 1 or 2.

5. A parathyroid hormone production suppressing agent described in the Claim 1 wherein m of the formula (1) is 1, q is 0, r is 1 and X is oxygen atom.

6. A remedy for hyperparathyroidism described in the Claim 2 wherein m of the formula (1) is 1, q is 0, r is 1 and X is oxygen atom.

7. A parathyroid hormone production suppressing agent described in the Claim 1 wherein m of the formula (1) is 1, q is 0, r is 1 and X is carbon atom.

8. A remedy for hyperparathyroidism described in the Claim 2 wherein m of the formula (1) is 1, q is 0, r is 1 and X is carbon atom.

9. A parathyroid hormone production suppressing agent described in the Claim 1 wherein m of the formula (1) is 2, q is 0, r is 1 and X is oxygen atom.

10. A remedy for hyperparathyroidism described in the Claim 2 wherein m of the formula (1) is 2, q is 0, r is 1 and X is oxygen atom.

11. A parathyroid hormone production suppressing agent described in the Claim 1 wherein m of the formula (1) is 1, q is 1, r is 0 and X is carbon atom.

12. A remedy for hyperparathyroidism described in the Claim 2 wherein m of the formula (1) is 1, q is 1, r is 0 and X is carbon atom.
